# FASCICULE DE BREVET EUROPEEN

(11) **EP 0 637 600 B1**
(45) Date de publication et mention de la délivrance du brevet: **09.04.1997**
(21) Numéro de dépôt: 94401808.4
(22) Date de dépôt: 04.08.1994
(51) Int. Cl.: C08G 18/08, C08G 18/42, A61K 7/043, A61K 7/48

(54) **Nouveaux polyester-polyuréthannes, leur procédé de préparation, pseudo-latex réalisés à partir desdits polyester-polyuréthannes et leur utilisation dans des compositions cosmétiques**
Neue Polyester-Polyurethane, ihr Herstellungsverfahren, aus diesen Polyester-Polyurethanen hergestellter Pseudolatex sowie dessen Verwendung in kosmetischen Zusammensetzungen
New polyester polyurethanes, their preparation process, pseudo-latex prepared from these polyester polyurethanes and their use in cosmetic compositions

(30) Priorité: 04.08.1993 FR 9309608
(43) Date de publication de la demande: 08.02.1995
(73) Titulaire: L'OREAL, 75008 Paris (FR)
(72) Inventeur: Mondet, Jean, F-93700 Drancy (FR); Lion, Bertrand, F-93190 Livry-Gargan (FR); Mougin, Nathalie, F-75010 Paris (FR); de la Poterie, Valérie, F-77820 Le Chatelet en Brie (FR); Piot, Bertrand, F-92250 La Garenne-Colombes (FR)
(74) Mandataire: Stalla-Bourdillon, Bernard

(56) Documents cités:
- EP-A- 0 076 956
- FR-A- 1 457 975
- US-A- 3 975 350

## Description

La présente invention a pour objet une nouvelle famille de polyester-polyuréthannes, leur procédé de préparation, les pseudo-latex réalisés à l'aide desdits polyester-polyuréthannes ainsi que des compositions cosmétiques contenant lesdits pseudo-latex.

Il est d'usage courant d'utiliser dans de nombreuses formulations cosmétiques, et notamment dans différents produits de maquillage tels que des vernis à ongles, des mascaras et des eye-liners, à titre de résine filmogène, des polyuréthannes. La résine, pour être satisfaisante, doit non seulement présenter de bonnes propriétés filmogènes, mais également de bonnes propriétés de rémanence, c'est-à-dire être difficilement éliminable de son support par simple lavage à l'eau ou à l'aide de détergents.

Il a été décrit dans la demande de brevet EP 418.469, des compositions de vernis à ongles contenant à titre de résine filmogène des dispersions aqueuses de polyuréthannes aliphatiques.

Il a également été décrit dans la demande de brevet EP 391.322, des vernis à ongles contenant une dispersion aqueuse d'un polyuréthanne et/ou d'un polyuréthanne copolymère.

L'utilisation de ces résines ne permet pas cependant de conduire à des compositions présentant de bonnes propriétés cosmétiques du fait notamment d'un manque de rémanence.

Il a maintenant été constaté de façon surprenante et inattendue qu'une nouvelle famille de polyester-polyuréthannes présentait non seulement de bonnes propriétés filmogènes, mais permettait également d'obtenir des films possédant à la fois une grande rigidité et une excellente rémanence à l'eau et aux détergents.

Ces excellentes propriétés ont pu être obtenues grâce au choix particulier d'un α,ω-dihydroxypolyester entrant dans la synthèse des polyester-polyuréthannes selon l'invention.

Les polyester-polyuréthannes selon l'invention permettent la préparation de pseudo-latex également très rémanents et, de plus, particulièrement stables sans utilisation de tensioactifs additionnels, dans la mesure où ils comportent en outre des fonctions ioniques.

La présente invention a pour objet à titre de produit industriel nouveau, un polyester-polyuréthanne contenant des motifs répondant aux formules (I) et (II) suivantes : 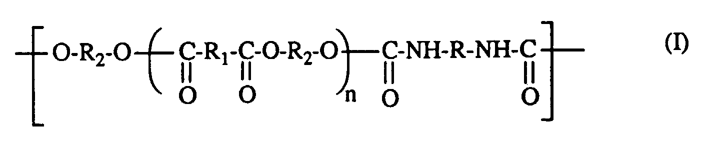 dans laquelle :
R représente un radical alkylène, cycloalkylène ou un radical aromatique divalent, ayant de 6 à 15 atomes de carbone,
n représente un nombre entier tel que le poids moléculaire de l'unité répétitive est compris entre 400 et 5.000,
R₁ représente un radical divalent choisi dans le groupe constitué par :
   (i) (̵CH₂ 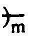 , m étant un nombre entier compris entre 2 et 12, et
   (ii) 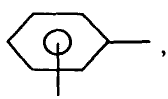 la liaison mobile étant en position ortho, méta ou para,
R₂ représente un radical divalent choisi dans le groupe constitué par : 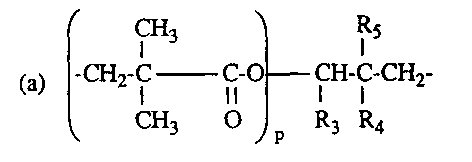
R₃ représentant un atome d'hydrogène ou un radical alkyle ramifié, ayant de 1 à 3 atomes de carbone,
R₄ représentant un atome d'hydrogène ou un radical alkyle, linéaire ou ramifié, ayant de 1 à 4 atomes de carbone,
R₅ représentant un radical alkyle, linéaire ou ramifié, ayant de 1 à 4 atomes de carbone, et
p étant 0 ou 1, 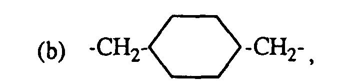 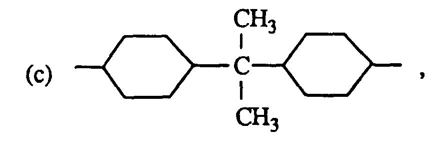 et 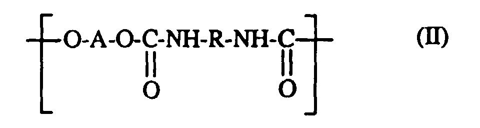 dans laquelle :
   R est tel que défini ci-dessus pour les motifs de formule (I),

A représente un radical alkylène ayant de 2 à 20 atomes de carbone substitué par une fonction acide carboxylique ou sulfonique, sous forme salifiée ou non, ou interrompu par un atome d'azote tertiaire,
le rapport molaire entre les motifs (II) et (I) étant compris entre 1 et 10, et de préférence entre 1 et 5.

Le radical divalent R du motif de formule (I) est de préférence choisi dans le groupe constitué par les radicaux hexaméthylène, 4,4'-biphénylèneméthane, 2,4- et/ou 2,6-tolylène, 1,5-naphtylène, p-phénylène, méthylène 4,4'-biscyclohexyle et le radical divalent dérivé de l'isophorone.

Le radical divalent A du motif de formule (II) est de préférence choisi dans le groupe constitué par : 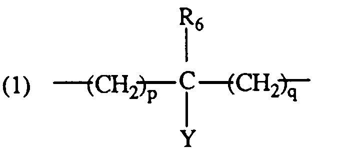
R₆ représentant un radical alkyle, linéaire ou ramifié, ayant de 1 à 3 atomes de carbone,
Y représentant un groupe acide carboxylique ou acide sulfonique, et
p et q, identiques ou différents, représentant un nombre entier compris entre 1 et 5, ou un sel desdits acides, 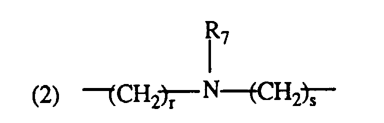
R₇ représentant un radical alkyle, linéaire ou ramifié, ayant de 1 à 4 atomes de carbone, et
r et s, identiques ou différents, représentant un nombre entier compris entre 1 et 10,

Les polyester-polyuréthannes selon l'invention peuvent en outre contenir des motifs répondant à la formule (III) suivante : 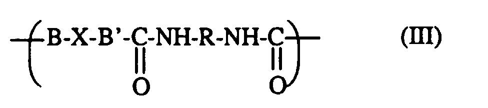 dans laquelle :
R est tel que défini ci-dessus pour les motifs de formule (I),
B et B', identiques ou différents, représentent -O- ou -NH-, B et B' ne pouvant simultanément représenter -O-, et
X représente un radical alkylène ou cycloalkylène ayant de 2 à 12 atomes de carbone ou un radical aromatique divalent ayant de 6 à 12 atomes de carbone,
ledit motif étant présent en une proportion telle que le rapport molaire de la somme des motifs des formules (II) et (III) et des motifs de formule (I) est un nombre entier compris entre 1 et 10 et de préférence entre 1 et 5.

Le poids moléculaire des polyester-polyuréthannes selon l'invention, mesuré par chromatographie d'exclusion stérique, est généralement compris entre 4.000 et 500.000 et de préférence compris entre 6.000 et 200.000.

La présente invention a également pour objet le procédé de préparation desdits polyester-polyuréthannes. Ce procédé consiste à faire réagir, dans un solvant organique, un α,ω-dihydroxypolyester répondant à la formule (IV) suivante : 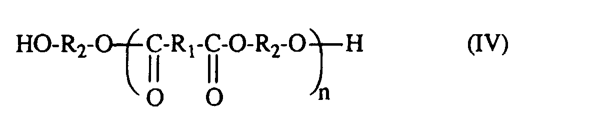 dans laquelle :
R₁, R₂ et n sont tels que définis ci-dessus pour les motifs de formule (I)
avec un excès d'un diisocyanate répondant à la formule (V) suivante :

O=C=N-R-N=C=O (V)

dans laquelle R est tel que défini ci-dessus pour les motifs de formule (I), puis à coupler les chaînes du polyester-polyuréthanne obtenu précédemment par un diol répondant à la formule (VI) suivante :

HO-A-OH (VI)

dans laquelle :
A est tel que défini ci-dessus pour les motifs de formule (II), à une température comprise entre 40 et 100°C en présence d'un sel d'étain en tant que catalyseur.

Le solvant organique utilisé dans le procédé selon l'invention est de préférence choisi dans le groupe constitué par l'acétone, la méthyléthylcétone, le tétrahydrofuranne et le 1,2-dichloroéthane, ces solvants étant inertes vis-à-vis des groupes isocyanates.

Le sel d'étain est de préférence choisi parmi l'éthyl-2 hexanoate d'étain et le dibutyl dilaurate d'étain.

L'α,ω-dihydroxypolyester de formule (IV) utilisé en tant que produit de départ dans la synthèse des polyester-polyuréthannes selon l'invention a de préférence un poids moléculaire compris entre 400 et 5.000.

Parmi les α,ω-dihydroxypolyesters de formule (IV) particulièrement préférés, on peut mentionner celui dans lequel R₂ représente un radical divalent de formule : 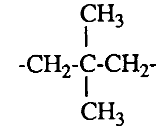 et R₁ représente -(CH₂)₈- ou le radical p-phénylène.

Le diisocyanate de formule (V) utilisé dans le procédé selon l'invention est de préférence choisi dans le groupe constitué par le diphénylméthane 4,4'-diisocyanate et le dicyclohexylméthane 4,4'-diisocyanate (ou dicyclohexylméthylène 4,4'-diisocyanate).

Le diol de formule (VI) utilisé dans le procédé selon l'invention est de préférence choisi dans le groupe constitué par l'acide diméthylol propionique et la N-méthyl-diéthanolamine.

Selon un mode de réalisation particulier du procédé selon l'invention, on fait réagir en outre un coupleur répondant à la formule (VII) suivante :

H-B-X-B'-H (VII)

dans laquelle :
B, B' et X sont tels que définis ci-dessus pour les motifs de formule (III).

De préférence, celui-ci est choisi dans le groupe constitué par le 1,3-diaminopropane et l'éthanolamine.

Le polyester-polyuréthanne obtenu selon le procédé décrit précédemment peut être éventuellement purifié par exemple par précipitation dans un solvant non polaire tel que le cyclohexane.

La présente invention a également pour objet, à titre de produit industriel nouveau, un pseudo-latex stable constitué de particules de polyester-polyuréthanne tel que défini et obtenu précédemment, neutralisé à l'aide d'un agent neutralisant qui peut être soit une base minérale ou organique lorsque le radical A des motifs de formule (II) est substitué par une fonction acide carboxylique ou sulfonique, soit un acide minéral ou organique lorsque le radical A des motifs de formule (II) est interrompu par un atome d'azote tertiaire, à un taux de neutralisation compris entre 20 et 100 %, le diamètre moyen des particules étant compris entre 5 et 300 nm.

On entend, selon l'invention, par l'expression "pseudo-latex" une suspension constituée de particules généralement sphériques du polyester-polyuréthanne, celles-ci étant obtenues par dispersion du polyester-polyuréthanne dans une phase aqueuse appropriée. L'expression "pseudo-latex" ne doit pas être confondue avec l'expression "latex" ou "latex synthétique" qui est également une suspension constituée de particules d'un polymère, mais dont lesdites particules sont obtenues directement par polymérisation d'un ou plusieurs monomères dans une phase aqueuse appropriée.

Les pseudo-latex selon l'invention sont obtenus selon les méthodes connues de préparation des pseudo-latex sous réserve, toutefois, de certaines particularités qui seront mentionnées ci-après.

Le procédé conventionnel de préparation des pseudo-latex consiste à dissoudre un polymère insoluble dans l'eau, dans un solvant organique, soluble ou partiellement soluble dans l'eau, à disperser sous agitation la dispersion ainsi obtenue dans de l'eau et à procéder ensuite à l'élimination du solvant organique par évaporation sous vide, ce qui conduit à une suspension constituée de particules du polymère dont la taille est généralement inférieure au micromètre.

Selon ce procédé général, l'emploi d'un tensioactif, d'un mélange de tensioactifs ou d'un polymère colloïde protecteur ou encore d'un mélange tensioactifs/polymère colloïde protecteur est indispensable en vue d'obtenir une bonne stabilisation des particules.

Par contre, les polyester-polyuréthannes selon l'invention comportant des fonctions ioniques, partiellement ou totalement neutralisées, permettent d'obtenir des pseudo-latex particulièrement stables en l'absence de stabilistant hydrophile, de tensioactif ou de colloïde protecteur.

Il va de soi que la nature acide ou basique de l'agent neutralisant qu'il conviendra d'utiliser pour neutraliser le polyester-polyuréthanne sera fonction de la nature des fonctions ioniques portées par ledit polyester-polyuréthanne.

Lorsque le polyester-polyuréthanne comporte une fonction acide carboxylique ou sulfonique, l'agent neutralisant peut être une base minérale telle que la soude, la potasse ou l'ammoniaque, ou une base organique telle qu'un aminoalcool choisi parmi le 2-amino 2-méthyl 1-propanol (AMP), la triéthanolamine, la triisopropanolamine (TIPA), la monoéthanolamine, la diéthanolamine, la tri[(2-hydroxy)1-propyl]amine, le 2-amino 2-méthyl 1,3-propanediol (AMPD) et le 2-amino 2-hydroxyméthyl 1,3-propanediol ou bien une diamine telle que la lysine.

Lorsque le polyester-polyuréthanne comporte une fonction amine tertiaire, l'agent neutralisant peut être un acide minéral tel que l'acide chlorhydrique ou un acide organique tel que l'acide lactique, l'acide glycolique ou l'acide mandélique.

La neutralisation peut être réalisée soit *in situ* dans la solution du polyester-polyuréthanne dans le solvant organique par addition de la quantité déterminée d'agent neutralisant, soit lors de la préparation de l'émulsion, l'agent neutralisant se trouvant alors dans la phase aqueuse de l'émulsion. Le solvant organique utilisé doit être un solvant volatil ou un mélange de tels solvants présentant un point d'ébullition inférieur à celui de l'eau et être miscible ou partiellement miscible à l'eau.

Le solvant organique tel que défini ci-dessus est de préférence choisi parmi l'acétone, la méthyléthylcétone, le tétrahydrofuranne, l'acétate de méthyle, l'acétate d'éthyle, l'isopropanol et l'éthanol.

Après l'obtention du polyester-polyuréthanne totalement ou partiellement neutralisé dans le solvant organique, on procède alors à la préparation d'une émulsion en versant, sous agitation, à la solution organique obtenue, une quantité appropriée d'eau contenant éventuellement un agent anti-mousse dont le rôle sera de faciliter l'évaporation ultérieure de la phase organique.

Selon une variante du procédé tel que défini ci-dessus, la neutralisation des fonctions ioniques du polyester-polyuréthanne, en solution dans un solvant organique, peut être réalisée lors de la formation de l'émulsion en versant une solution aqueuse contenant la quantité requise de l'agent neutralisant.

Lors de la formation de l'émulsion, l'agitation est de préférence réalisée à l'aide d'un disperseur cisaillant du type Moritz ou Ultra Turrax ou Raineri équipé de pales défloculantes.

L'émulsion ainsi obtenue est particulièrement stable sans qu'il soit nécessaire d'employer un agent tensioactif dans la mesure où les groupes ioniques du polyester polyuréthanne se placent à l'interface avec l'eau et protègent les gouttelettes de la coalescence par répulsion électrostatique.

Après formation de l'émulsion à une température comprise entre la température ambiante et 70°C environ, on procède alors à l'évaporation sous pression réduite du solvant organique jusqu'à son élimination totale, l'évaporation étant de préférence réalisée sous léger chauffage.

On obtient ainsi un pseudo-latex, c'est-à-dire une dispersion aqueuse de particules du polyester-polyuréthanne filmogène, qui est exempte de tout tensioactif ou de tout autre stabilisant hydrophile, tout en étant très stable.

La taille moyenne des particules du pseudo-latex et leur polydispersité peuvent être réglées en faisant varier, lors de la préparation dudit pseudo-latex, les proportions respectives entre le polyester-polyuréthanne, le solvant organique et l'eau, modifiant ainsi notamment la viscosité dudit pseudo-latex et la brillance du film obtenu après évaporation. La taille moyenne des particules dépent également du taux de neutralisation et de la nature du neutralisant.

Selon un mode de réalisation préféré des pseudo-latex selon l'invention, la taille moyenne des particules est comprise entre 10 et 250 nm.

La polydispersité en taille des particules, mesurée en diffusion quasi-élastique de lumière, est généralement inférieure à 0,5 et de préférence comprise entre 0,05 et 0,4.

Les polyester-polyuréthannes selon l'invention peuvent être plastifiés pour améliorer la formation des films à température ambiante. La plastification peut être réalisée par mélange du pseudo-latex de polyester-polyuréthanne selon l'invention avec une dispersion aqueuse d'un polyéther-polyuréthanne ou d'un polyester-polyuréthanne à caractère élastomère, ces dispersions étant de même nature ionique que le polyester-polyuréthanne selon l'invention.

Comme dispersions de polyéther-polyuréthannes à caractère élastomère et de nature anionique, on peut citer notamment celles vendues sous les dénominations de "Sancure 861" ou de "Sancure 878" par la Société Sanncor, ou sous la dénomination de "Neorez-R970" par la Société ICI.

Comme dispersions de polyester-polyuréthannes à caractère élastomère, et de nature anionique, on peut notamment citer celle vendue sous la dénomination de "Neorez-R974" par la Société ICI.

La plastification peut être également réalisée à l'aide de plastifiants classiques non polymériques. Il est alors nécessaire que le plastifiant soit un bon solvant du polyester-polyuréthanne selon l'invention et soit de préférence insoluble dans l'eau. Parmi les plastifiants hydrophobes, on peut citer notamment :
- les phtalates et adipates de diéthyle, de dibutyle et de 2-diéthylhexyle,
- les tartrates de diéthyle et de dibutyle,
- les phosphates de diéthyle, de dibutyle et de 2-diéthylhexyle,
- les dérivés de propylèneglycol choisis parmi le propylèneglycol phényléther, le propylèneglycol diacétate, le dipropylèneglycol butyléther, le tripropylèneglycol butyléther,
- les esters de glycérol tels que le triacétate de glycérol (triacétine),
- le monophényléther de propylèneglycol vendu sous la dénomination de "Dowanol PPH" par la Société Dow Chemical et le dipropylèneglycol n-butyléther vendu sous la dénomination de "Dowanol DPnB" par la Société Dow Chemical.

Le plastifiant peut être incorporé soit après la réalisation du pseudo-latex, soit pendant la réalisation du pseudo-latex lors de la formation de l'émulsion, le plastifiant étant alors incorporé dans la phase organique de l'émulsion.

L'invention a également pour objet une composition cosmétique contenant, dans un support cosmétique approprié, un pseudo-latex tel que défini ci-dessus.

La proportion en pseudo-latex dans les compositions cosmétiques est généralement comprise entre 0,5 et 30 %, et de préférence entre 1 et 25 % en poids par rapport au poids total de la composition.

Les compositions cosmétiques selon l'invention peuvent se présenter sous diverses formes, par exemple sous forme de produits de maquillage pour les ongles ou les cils tels que des vernis à ongles ou des mascaras, des produits de soin de la peau tels que des masques ou des sérums.

Les compositions selon l'invention peuvent également se présenter sous forme de produits de soin des cheveux tels que des shampooings coiffants, des lotions de traitement des pointes des cheveux, des vernis à cheveux et des gels coiffants.

Les compositions selon l'invention peuvent en outre contenir des filtres solaires UV-A ou UV-B ou à bande large et être utilisées comme produits anti-solaires.

Les compositions selon l'invention peuvent contenir par ailleurs des adjuvants cosmétiques conventionnels choisis parmi des corps gras, des solvants organiques, des silicones, des agents épaississants, des adoucissants, des agents anti-mousse, des agents hydratants, des humectants, des agents durcisseurs des ongles, des polymères anioniques, non-ioniques ou amphotères ou leurs mélanges, des antiperspirants, des agents alcalinisants, des colorants, des pigments et des agents propulseurs lorsque les compositions se présentent sous forme aérosol.

Plus précisément, comme corps gras, on peut utiliser une huile ou une cire ou leurs mélanges, des acides gras, des alcools gras, des esters d'acides gras tels que les triglycérides d'acides gras en C₆ à C₁₈, de la vaseline, de la paraffine, de la lanoline, de la lanoline hydrogénée ou acétylée.

Parmi les huiles, on peut citer les huiles minérales, animales, végétales ou les huiles de synthèse et notamment l'huile de vaseline, de paraffine, de ricin, de jojoba, de sésame, ainsi que les huiles et les gommes de silicones et les isoparaffines.

Parmi les cires animales, fossiles, végétales, minérales ou de synthèse, on peut notamment citer la cire d'abeille, de caroube, de Candelilla, l'ozokérite, les cires microcristallines ainsi que les cires et les résines de silicone.

Parmi les agents épaississants, on peut citer :
- les dérivés de celluloses tels que l'hydroxyéthylcellulose, la méthylcellulose, l'hydroxypropylcellulose et la carboxyméthylcellulose. Parmi ceux-ci, on peut citer notamment les gommes vendues sous la dénomination de "Cellosize QP 4400H" par la Société Amerchol,
- la gomme de caroube, la gomme de guar, la gomme de guar quaternisée vendue sous la dénomination de "Jaguar C-13-S" par la Société Meyhall, la gomme d'hydroxypropylguar, la gomme de xanthane,
- les acides polyacryliques réticulés tels que ceux vendus sous la dénomination de "Carbopol" par la Société Goodrich,
- les copolymères acide acrylique/acrylate d'alkyle (C₁₀/C₃₀) réticulés tels que ceux vendus sous les dénominations de "Pemulen TR₁" et de "Pemulen TR₂" par la Société Goodrich,
- les polymères poly(méth)acrylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et copolymères réticulés d'acrylamide vendus sous les dénominations de "PAS 5161" ou "Bozepol C" par la Société Hoechst, de "Sepigel 305" par la Société Seppic ou de "Salcare SC92" par la Société Allied Colloid, ou encore
- les homopolymères réticulés de chlorure de méthacryloyloxyéthyltriméthylammonium vendus sous la dénomination de "Salcare SC95" par la Société Allied Colloid.

Les compositions selon l'invention trouvent tout particulièrement une application dans les soins et le traitement des ongles.

Ainsi, celles-ci peuvent constituer une sous-couche pour l'application ultérieure après séchage d'un vernis à ongles coloré classique. La sous-couche protège ainsi l'ongle de l'effet agressif du mélange solvant du vernis à ongles et empèche par ailleurs que la kératine de l'ongle soit colorée sous l'effet des pigments. Cette sous-couche permet par ailleurs d'améliorer l'adhésion du vernis à ongles et peut contenir divers actifs pour les soins des ongles dans la mesure où ceux-ci sont solubles dans l'eau ou facilement dispersables.

L'intérêt d'utiliser, selon ce mode de réalisation, une sous-couche d'une composition selon l'invention permet de rendre pelable le vernis à ongles par enlèvement simultané de la sous-couche et de la couche de vernis à ongles.

Les compositions selon l'invention peuvent également constituer une couche de revêtement (ou "top coat") après application et séchage d'un vernis à ongles coloré classique. Cette couche supérieure apporte de la brillance et une meilleure résistance mécanique.

Selon une forme particulièrement préférée, les compositions selon l'invention se présentent sous forme de vernis à ongles aqueux coloré.

Ces vernis à ongles contiennent au moins un pseudo-latex tel que défini ci-dessus en une proportion de 1 à 25 % en poids, au moins un agent épaississant en une proportion de 0,01 à 5 % et de préférence de 0,1 à 1 % en poids, au moins un pigment en une proportion inférieure à 3 % et de préférence comprise entre 0,5 et 2 % en poids et au moins un agent mouillant en une proportion de 0,1 à 1 % en poids, le reste étant essentiellement constitué par de l'eau.

On va maintenant donner à titre d'illustration de l'invention plusieurs exemples de préparations des polyester-polyuréthannes ainsi que de pseudo-latex et de compositions cosmétiques les contenant.

### Procédé de préparation des α,ω-dihydroxypolyesters

### EXEMPLE A : Préparation d'un α,ω-dihydroxypolyester 50/50 en moles de téréphtalatelsébaçate de 2,2-diméthyl 1,3-propane diol

Dans un réacteur de 500 ml, on introduit 274,6 g (2,64 moles) de 2,2-diméthyl 1,3-propanediol et 138 g (0,6 mole) de sébaçate de diméthyle préalablement fondu, puis chauffe sous agitation jusqu'à obtention d'un milieu limpide, c'est à dire à environ 100°C. On ajoute alors 116,4 g (0,6 mole) de téréphtalate de diméthyle, puis chauffe à 150°C et ajoute alors 1,6 g (0,3 % en poids par rapport au poids total des réactifs) d'acétate de zinc dihydrate.

On maintient la température du milieu réactionnel à 150°C durant 3 heures afin d'éliminer le méthanol formé par la transestérification puis l'élève à 200°C en 45 minutes et on la maintient durant 3 heures.

On laisse ensuite revenir à température ambiante en diminuant l'agitation. Dès que la température atteint 50°C, on ajoute alors 300 ml de 1,2-dichloroéthane.

On dilue ensuite la solution dans 1,7 l de 1,2-dichloroéthane puis la purifie par lavage à l'eau.

La phase organique est ensuite séchée sur sulfate de sodium anhydre puis, après filtration, le solvant d'extraction est éliminé par évaporation sous vide.

On obtient ainsi 300 g d'α,ω-dihydroxypolyester attendu se présentant sous forme d'une pâte à température ambiante.

Caractéristiques de l'α,ω-dihydroxypolyester obtenu :
Indice d'hydroxyle : 190
Poids moléculaire (déterminé d'après l'indice d'hydroxyle) : 560
IR et RMN : conformes aux attendus.

### EXEMPLES B à D:

Selon le même procédé que décrit à l'exemple A, on a préparé les α,ω-dihydroxypolyesters du Tableau I suivant :

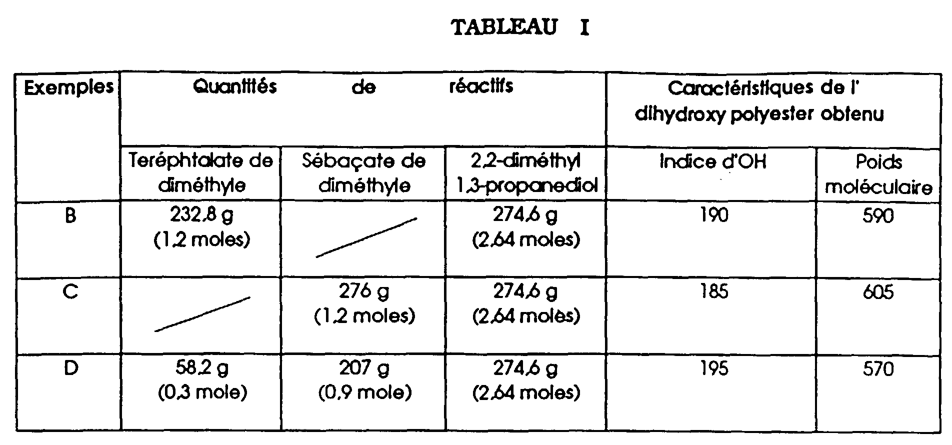

### Procédé de préparation des polyester-polyuréthannes

### EXEMPLE 1 :Préparation d'un polyester-polyuréthanne à partir du prépolymère de l'exemple A

Dans un réacteur d' 1 l, on introduit sous azote 78,6 g (0,3 mole) de dicyclohexylméthane diisocyanate et 150 g de 1,2-dichloroéthane. En 30 minutes, sous courant d'azote, on porte le mélange au reflux puis laisse sous agitation 30 minutes à 80°C.

On ajoute alors goutte à goutte en 15 minutes et à 80°C, 83,2 g (0,15 mole) du α,ω-dihydroxypolyester de l'exemple A préalablement dissous dans 200 g de 1,2-dichloroéthane.

Après 3 heures, on ajoute 4,8 g (0,075 mole) d'éthanolamine en solution dans 50 g de 1,2-dichloroéthane et on laisse réagir 1 heure. Puis on introduit 10,05 g (0,075 mole) d'acide diméthylol propionique préalablement dissous dans 30 g de diméthylformamide, et ensuite 0,45 g de 2-éthyl hexanoate d'étain.

Après 12 heures à 80°C, on vérifie par infrarouge, l'absence de groupes isocyanates résiduels. Si la consommation de dicyclohexylméthane diisocyanate n'est pas totale, on ajoute au mélange 100 g d'éthanol et laisse réagir 2 heures au reflux.

Après refroidissement, on purifie la solution de polyester-polyuréthanne par précipitation dans du cyclohexane, et sèche le précipité sous vide en étuve à 50°C.

Caractéristiques du polyester-polyuréthanne obtenu :
Rendement: : 91 %
Dosage des groupes acides par potentiométrie : 6,4 % en poids (théorie : 5,7 %)
Poids moléculaire (chromatographie d'exclusion stérique - éluant : tétrahydrofuranne) : 9000

### EXEMPLES 2 à 10 :

Selon le même procédé que décrit à l'exemple 1, on a préparé les polyester-polyuréthannes du Tableau II suivant :

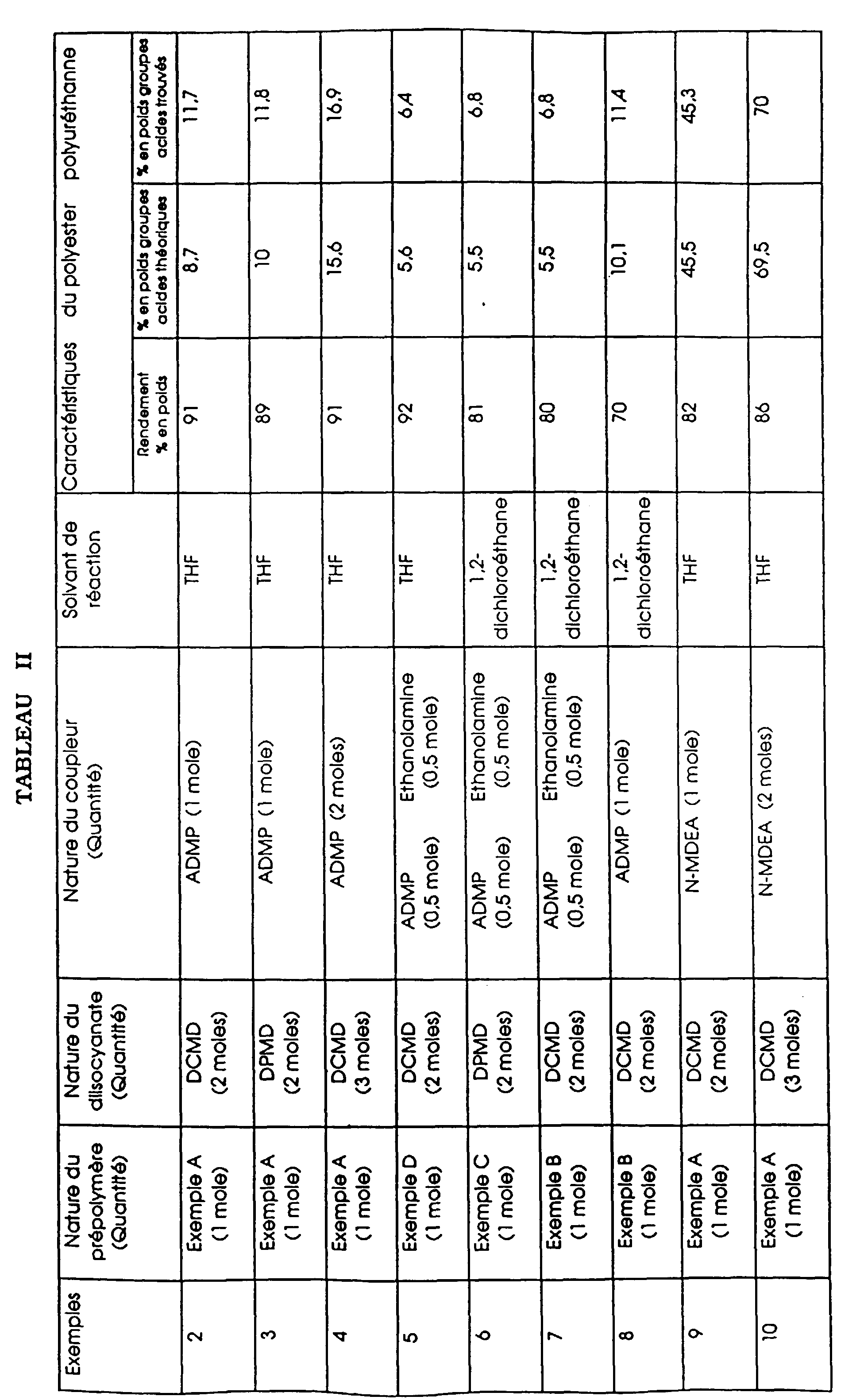

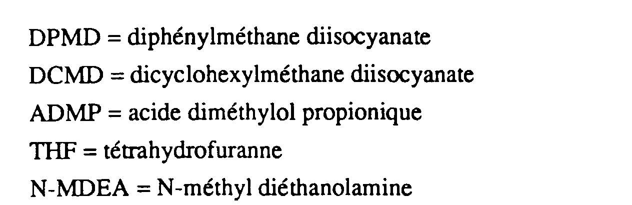

### EXEMPLE 11 :

Selon le même procédé que décrit à l'exemple 1, on a fait réagir les réactifs suivants:
- dicyclohexylméthane diisocyanate : 78,6 g (0,3 mole)
- α,ω-dihydroxypolyester de l'exemple A : 83,2 g (0,15 mole)

Après 3 heures, on refroidit le mélange à 5°C, puis ajoute 3,3 g (0,045 mole) de 1,3-diaminopropane préalablement mis en solution dans 50 g de tétrahydrofuranne. Après 1 heure sous agitation, on chauffe à 80°C et ajoute 14,07 g (0,105 mole) d'acide diméthylol propionique et 0,45 g de 2-éthyl hexanoate d'étain.

Après 12 heures à 80°C, on vérifie par infrarouge, l'absence de groupes isocyanates résiduels. Si la consommation de dicyclohexylméthane diisocyanate n'est pas totale, on ajoute au mélange 100 g d'éthanol, et laisse réagir 2 heures au reflux.

Après refroidissement, on purifie la solution du polyester-polyuréthanne par précipitation dans du cyclohexane puis sèche le précipité sous vide en étuve à 50°C.

Caractéristiques du polyester-polyuréthanne obtenu :
Rendement : 93 %
Dosage des groupes acides par potentiométrie : 8,5 % en poids (théorie : 7,2 %).

### Préparation des pseudo-latex des polyester-polyuréthannes

### EXEMPLE I : Préparation du pseudo-latex du polyester-polyuréthanne de l'Exemple 1

A température ambiante, on dissout 10 g du polyester-polyuréthanne obtenu à l'exemple 1 dans 100 g de tétrahydrofuranne, agite le mélange avec un agitateur de type Moritz puis introduit progressivement un mélange de 45 g d'eau et 0,41 g d'amino-2 méthyl-2 propanol et agite 15 minutes.

On concentre alors l'émulsion ainsi obtenue à l'évaporateur rotatif sous vide partiel et à une température inférieure à 45°C, jusqu'à élimination totale du tétrahydrofuranne.

Le pseudo-latex de polyester-polyuréthanne ainsi obtenu est stable.

Caractéristiques du pseudo-latex obtenu :
Taux de neutralisation : 100 %
Concentration en polymères dans le pseudo-latex : 27 %
Taille moyenne des particules (mesurée par diffusion quasi-élastique de lumière à l'aide du Coulter N4 de la Société Coultronix : 50 nm
Polydispersité des particules : 0,3

### EXEMPLES II à XVII :

Selon le même procédé que décrit à l'exemple I, on a préparé les pseudo-latex de polyester-polyuréthannes du Tableau III suivant :

**TABLEAU III**

| Exemples | Nature du polyester-polyuréthanne | Natue du neutralisant et taux de neutralisation | | Extrait sec final % | Taille moyenne des particules nm | Polydispersité de taille des particules |
|---|---|---|---|---|---|---|
| II | Exemple 2 | NaOH | 100 % | 30,4 | 50 | 0,23 |
| III | Exemple 2 | AMP | 100 % | 24 | 45 | 0,25 |
| IV | Exemple 2 | NaOH + L-lysine | 50 % 25 % | 27 | 110 | 0,30 |
| V | Exemple 3 | AMP | 100 % | 23 | 30 | 0,23 |
| VI | Exemple 4 | AMP | 60 % | 30 | 30 | 0,27 |
| VII | Exemple 4 | NaOH + L-lysine | 50 % 25 % | 30 | 120 | 0,23 |
| VIII | Exemple 5 | AMP | 100 % | 23 | 50 | 0,33 |
| IX | Exemple 6 | AMP | 100 % | 25 | 45 | 0,30 |
| X | Exemple 7 | AMP | 100 % | 23 | 25 | 0,28 |
| XI | Exemple 8 | AMP | 100 % | 30 | 30 | 0,25 |
| XII | Exemple 8 | NaOH + L-lysine | 50 % 25 % | 25 | 100 | 0,25 |
| XIII | Exemple 9 | HCl | 80 % | 25 | 30 | <0,2 |
| XIV | Exemple 9 plastifié* | HCl | 80 % | 25 | 30 | 0,30 |
| XV | Exemple 10 | HCl | 70 % | 25 | 25 | <0,3 |
| XVI | Exemple 11 | AMP | 100 % | 23 | 40 | 0,25 |
| XVII | Exemple 11 | NaOH + L-lysine | 50 % 25 % | 22 | 130 | 0,30 |

| | | | | | | |
|---|---|---|---|---|---|---|
| * la plasticifation est réalisée avec 5 % de phényléther de propylène glycol ayant été introduit dans la phase organique avant la réalisation de l'émulsion. | | | | | | |

### EXEMPLES DE COMPOSITIONS

Dans les exemples suivants, les lettres "MA" signifient "matière active" lorsque le produit utilisé, commercial ou non, se trouve sous forme d'une solution ou d'une dispersion dans un solvant.

### EXEMPLE 1 : Vernis à ongles

On prépare ce vernis à ongles en procédant au mélange des ingrédients suivants :
- Pseudo-latex de l'Exemple I 24,88 g (MA)
- Dipropylèneglycol n-butyléther vendu sous la dénomination de "Dowanol DPnB" par la Société Dow Chemical 3,23 g
- Epaississant associatif uréthanne non ionique vendu sous la dénomination de "SER AD FX 1100" par la Société Servo 0,3 g
- Pigments 1,0 g
- Tensio-actif fluoré vendu sous la dénomination de "Forafac 1157" par la Société Atochem 0,1 g
- Eau qsp 100 g

Le vernis à ongles obtenu est très résistant à l'eau : le film est intact après 1 heure sous agitation dans l'eau.

La dureté du film est mesurée par la méthode du pendule de Persoz, à 30°C et à 50 % d'humidité relative, après avoir laissé sécher ledit film d'une épaisseur de 300 µm sur une plaque de verre, dans une enceinte à 30°C et à 50 % d'humidité relative, et pendant 24 heures.

Dureté du film : 63,3 ± 2,5 secondes.

La dureté mesurée est très satisfaisante, le film adhère correctement à la kératine de l'ongle sans s'écailler. Il n'est pas collant et résiste aux rayures.

Le vernis obtenu selon l'invention s'applique facilement sur l'ongle et présente une brillance et une tenue satisfaisante.

### EXEMPLE 2 : Vernis à ongles

On prépare ce vernis à ongles en procédant au mélange des ingrédients suivants :
- Pseudo-latex de l'Exemple I 22,6 g (MA)
- Monophényléther de propylène glycol vendu sous la dénomination de "Dowanol PPH" par la Société Dow Chemical 2,49 g
- Epaississant associatif non ionique uréthanne vendu sous la dénomination de "SER AD FX 1100" par la Société Servo 0,3 g
- Pigments 1,0 g
- Tensio-actif fluoré vendu sous la dénomination de "Forafac 1157" par la Société Atochem 0,1 g
- Eau qsp 100 g

Le vernis à ongles obtenu est très résistant à l'eau : le film est intact après 1 heure sous agitation dans l'eau.

Dureté du film (30°C - 50 % humidité relative) : 77,0 ± 5,4 secondes.

### EXEMPLE 3 : Vernis à ongles

On prépare ce vernis à ongles en procédant au mélange des ingrédients suivants :
- Pseudo-latex de l'exemple IX 20,65 g (MA)
- Epaississant associatif non ionique uréthanne vendu sous la dénomination de "SER AD FX 1100" par la Société Servo 0,3 g
- Pigments 1,0 g
- Conservateurs 0,05 g
- Eau qsp 100 g

Dureté du film (30°C - 50 % humidité relative) : 89,4 ± 1,4 secondes.

Ce vernis s'applique facilement sur la surface des ongles. Le film obtenu présente une brillance satisfaisante et il résiste aux chocs.

La résistance du film à l'eau est très satisfaisante.

### EXEMPLE 4 : Vernis à ongles

On prépare ce vernis à ongles en procédant au mélange des ingrédients suivants :
- Pseudo-latex de l'Exemple XIII 20,7 g (MA)
- Monophényléther de propylène glycol vendu sous la dénomination de "Dowanol PPH" par la Société Dow Chemical 1,07 g
- Epaississant associatif non ionique uréthanne vendu sous la dénomination de "SER AD FX 1100" par la Société Servo 0,5 g
- Pigments 1,0 g
- Conservateurs 0,045 g
- Tensio-actif fluoré vendu sous la dénomination de "Forafac 1157" par la Société Atochem 0,3 g
- Eau qsp 100 g

Ce vernis s'applique facilement sur les ongles et présente une brillance et une dureté satisfaisantes.

Le vernis à ongles obtenu est très résistant à l'eau.

Dureté du film (30°C - 50 % humidité relative) : 104,90 ± 2,5 secondes.

### EXEMPLE 5 : Vernis à ongles

On prépare ce vernis à ongles en procédant au mélange des ingrédients suivants :
- Pseudo-latex de l'Exemple XV 20,7 g (MA)
- Epaississant associatif uréthanne non ionique vendu sous la dénomination de "SER AD FX 1100" par la Société Servo 0,5 g
- Pigments 1,0 g
- Conservateurs 0,045 g
- Tensio-actif fluoré vendu sous la dénomination de "Forafac 1157" par la Société Atochem 0,3 g
- Eau qsp 100 g

Ce vernis à ongles présente une très bonne résistance à l'eau.

Le film obtenu après séchage résiste aux chocs et aux rayures.

Dureté du film (30°C - 50 % humidité relative) : 232,3 ± 4,7 secondes.

### EXEMPLE 6 : Mascara

### Phase A :

- Stéarate de triéthanolamine 11,8 g
- Cire d'abeille 5 g
- Cire de carnauba 3 g
- Paraffine 1 g

### Phase B :

- Oxyde de fer noir 5 g

### Phase C :

- Gomme arabique 2 g
- Hydroxyéthylcellulose vendue sous la dénomination de "Cellosize QP" par la Société Amerchol 1,2 g

### Phase D :

- Pseudo-latex de l'Exemple I plastifié par 11 % de propylèneglycol phényléther vendu sous la dénomination de "Dowanol PPH" par la Société Dow Chemical 5 g
- Conservateurs qs
- Eau qsp 100 g

Ce mascara est obtenu en portant les ingrédients de la *Phase A* à 85°C, à laquelle on ajoute la *Phase B* et l'on agite à l'aide d'une turbine.

On porte ensuite l'eau de préparation à ébullition, on ajoute les conservateurs, puis à 85°C les ingrédients de la *Phase C.*

On ajoute alors la phase aqueuse obtenue (85°C) à la *Phase A* (80°C) sous agitation à l'aide d'une turbine (émulsification) puis on ajoute enfin, à 30°C, le pseudo-latex de la *Phase D* et agite à l'aide d'une pale.

### EXEMPLE 7 : Mascara

On prépare ce mascara, selon le même mode opératoire que celui décrit à l'exemple 6, ayant la composition suivante :

### Phase A :

- Stéarate de triéthanolamine 12 g
- Cire d'abeille 8 g
- Cire de carnauba 3 g
- Paraffine 2 g

### Phase B :

- Oxyde de fer noir 5 g

### Phase C :

- Gomme arabique 2,5 g
- Hydroxyéthylcellulose vendue sous la dénomination de "Cellosize QP" par la Société Amerchol 1,5 g
- Hydrolysat de kératine vendu sous la dénomination de "Kérasol" par la Société Croda 1 g

### Phase D :

- Pseudo-latex de l'Exemple I plastifié par 13 % de dipropylèneglycol n-butyléther vendu sous la dénomination de "Dowanol DPnB" par la Société Dow Chemical 4 g
- Conservateurs qs
- Eau qsp 100 g

### EXEMPLE 8: Mascara

On prépare ce mascara, selon le même mode opératoire que celui décrit à l'exemple 6, ayant la composition suivante :

### Phase A :

- Stéarate de triéthanolamine 11 g
- Cire d'abeille 10 g
- Cire de carnauba 2 g
- Paraffine 1 g

### Phase B :

- Oxyde de fer noir 6 g

### Phase C :

- Gomme arabique 0,8 g
- Hydroxyéthylcellulose vendue sous la dénomination de "Cellosize QP" par la Société Amerchol 2 g

### Phase D :

- Pseudo-latex de l'Exemple IX 6 g
- Conservateurs qs
- Eau qsp 100 g

### EXEMPLE 9: Mascara

On prépare ce mascara, selon le même mode opératoire que celui décrit à l'exemple 6, ayant la composition suivante :

### Phase A :

- Stéarate de glycérol 3 g
- Mélange d'esters d'acide laurique et de sorbitol et d'acide laurique et de sorbitol oxyéthyléné à 20 moles d'oxyde d'éthylène vendu sous la dénomination de "Tween 20" par la Société ICI 3,7 g
- Monoesters d'acide stéarique et de sorbitane vendu sous la dénomination de "Span 60" par la Société ICI 5,6 g
- Cire d'abeille 6 g
- Cire de carnauba 1,8 g
- Paraffine 7,8 g

### Phase B :

- Oxyde de fer noir 4,5 g

### Phase C :

- Hydroxyéthylcellulose vendue sous la dénomination de "Cellosize QP" par la Société Amerchol 1,5 g

### Phase D :

- Pseudo-latex de l'Exemple XV 2 g
- Conservateurs qs
- Eau qsp 100 g

### EXEMPLE 10 : Eye-liner

### Phase A :

- Oxyde de fer noir 12 g

### Phase B :

- Propylèneglycol 4 g
- Hydroxyéthylcellulose vendue sous la dénomination de "Cellosize QP" par la Société Amerchol 0,1 g
- Laponite 0,5 g

### Phase C :

- Pseudo-latex de l'Exemple I plastifié par 11 % de propylèneglycol phényléther vendu sous la dénomination de "Dowanol PPH" par la Société Dow Chemical 5 g
- Conservateurs qs
- Eau qsp 100 g

Cet eye-liner est obtenu en mélangeant les ingrédients de la *Phase B* à l'eau portée à 70°C dans laquelle les conservateurs ont été dissous.

On ajoute ensuite l'oxyde de fer noir de la *Phase A* et on mélange à l'aide d'une turbine à température ambiante puis ajoute le pseudo-latex de la *Phase C* sous agitation.

### EXEMPLE 11 : Lotion de mise en forme de la coiffure

On prépare cette lotion en procédant au mélange des ingrédients suivants :
- Pseudo-latex de l'exemple I 5 g (MA)
- Colorants qs
- Conservateurs qs
- Parfums qs
- Eau qsp 100 g

La lotion de mise en forme obtenue s'applique après un shampooing et confere un bon maintien de la coiffure.

### EXEMPLE 12 : Lotion de mise en forme de la coiffure

On prépare cette lotion en procédant au mélange des ingrédients suivants :
- Pseudo-latex de l'exemple I 1 g (MA)
- Monophényléther de propylène glycol vendu sous la dénomination de "Dowanol PPH" par la Société Dow Chemical 0,11 g
- Colorants qs
- Conservateurs qs
- Parfums qs
- Eau qsp 100 g

### EXEMPLE 13 : Lotion de coiffage en flacon pompe

On prépare cette lotion en procédant au mélange des ingrédients suivants :
- Pseudo-latex de l'exemple I 3 g (MA)
- Dipropylèneglycol n-butyléther vendu sous la dénomination de "Dowanol DPnB" par la Société Dow Chemical 0,39 g
- Colorants qs
- Conservateurs qs
- Parfums qs
- Eau qsp 100 g

La lotion obtenue est vaporisée sur la chevelure après un shampooing et confère un bon maintien à la coiffure.

### EXEMPLE 14 : Lotion de mise en forme de la coiffure

On prépare cette lotion en procédant au mélange des ingrédients suivants :
- Pseudo-latex de l'exemple IX 4 g (MA)
- Colorants qs
- Conservateurs qs
- Parfums qs
- Eau qs 100 g

La lotion de mise en forme obtenue s'applique facilement et apporte un maintien satisfaisant à la coiffure.

### EXEMPLE 15 : Lotion de coiffage en flacon pompe

On prépare cette lotion en procédant au mélange des ingrédients suivants :
- Pseudo-latex de l'exemple XIII 3 g (MA)
- Colorants qs
- Conservateurs qs
- Parfums qs
- Eau qsp 100 g

La lotion obtenue, appliquée sur les cheveux, donne une bonne tenue à la coiffure.

## Revendications

1. Polyester-polyuréthanne, caractérisé par le fait qu'il contient des motifs répondant aux formules (I) et (II) suivantes : 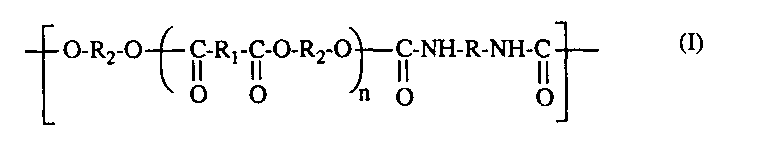 dans laquelle :
R représente un radical alkylène, cycloalkylène ou un radical aromatique divalent, ayant de 6 à 15 atomes de carbone,
n représente un nombre entier tel que le poids moléculaire de l'unité répétitive est compris entre 400 et 5.000,
R₁ représente un radical divalent choisi dans le groupe constitué par :
(i) (̵CH₂ 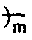 , m étant un nombre entier compris entre 2 et 12, et
(ii) 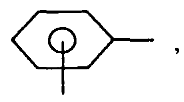 la liaison mobile étant en position ortho, méta ou para,
R₂ représente un radical divalent choisi dans le groupe constitué par : 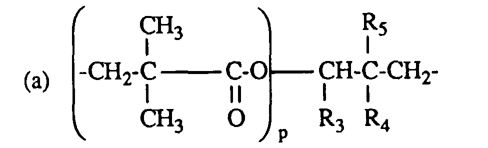
R₃ représentant un atome d'hydrogène ou un radical alkyle ramifié, ayant de 1 à 3 atomes de carbone,
R₄ représentant un atome d'hydrogène ou un radical alkyle, linéaire ou ramifié, ayant de 1 à 4 atomes de carbone,
R₅ représentant un radical alkyle, linéaire ou ramifié, ayant de 1 à 4 atomes de carbone, et
p étant 0 ou 1, 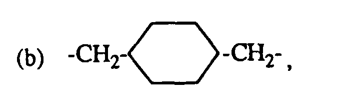 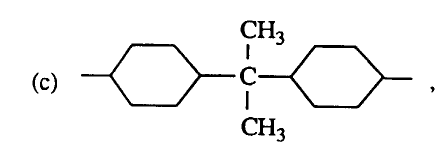 et 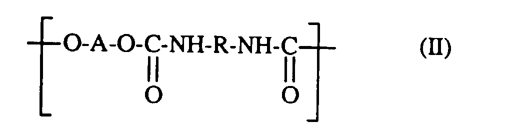
dans laquelle :
R est tel que défini ci-dessus pour les motifs de formule (I),
A représente un radical alkylène ayant de 2 à 20 atomes de carbone substitué par une fonction acide carboxylique ou sulfonique, sous forme salifiée ou non, ou interrompu par un atome d'azote tertiaire,
le rapport molaire entre les motifs (II) et (I) étant compris entre 1 et 10, et de préférence entre 1 et 5.

2. Polyester-polyuréthanne selon la revendication 1, caractérisé par le fait que le radical R du motif de formule (I) est choisi dans le groupe constitué par les radicaux hexaméthylène, 4,4'-biphénylèneméthane, 2,4- et/ou 2,6-tolylène, 1,5-naphtylène, p-phénylène, méthylène 4,4'-biscyclohexyle et le radical divalent dérivé de l'isophorone.

3. Polyester-polyuréthanne selon l'une quelconque des revendications précédentes, caractérisé par le fait que le radical divalent A du motif de formule (II) est choisi dans le groupe constitué par : 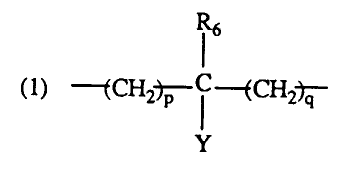
R₆ représentant un radical alkyle, linéaire ou ramifié, ayant de 1 à 3 atomes de carbone,
Y représentant un groupe acide carboxylique ou acide sulfonique, et
p et q, identiques ou différents, représentant un nombre entier compris entre 1 et 5, ou un sel desdits acides, 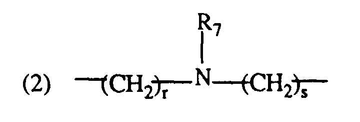
R₇ représentant un radical alkyle, linéaire ou ramifié, ayant de 1 à 4 atomes de carbone, et
r et s, identiques ou différents, représentant un nombre entier compris entre 1 et 10.

4. Polyester-polyuréthanne selon l'une quelconque des revendications précédentes, caractérisé par le fait qu'il contient en outre des motifs répondant à la formule (III) suivante : 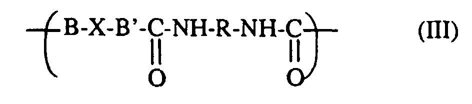 dans laquelle :
R est tel que défini à la revendication 1,
B et B', identiques ou différents, représentent -O- ou -NH-, B et B' ne pouvant simultanément représenter -O-, et
X représente un radical alkylène ou cycloalkylène ayant de 2 à 12 atomes de carbone ou un radical aromatique divalent ayant de 6 à 12 atomes de carbone,
ledit motif étant présent en une proportion telle que le rapport molaire de la somme des motifs des formules (II) et (III) et des motifs de formule (I) est un nombre entier compris entre 1 et 10 et de préférence entre 1 et 5.

5. Polyester-polyuréthanne selon l'une quelconque des revendications précédentes, caractérisé par le fait qu'il possède un poids moléculaire, mesuré par chromatographie d'exclusion stérique, compris entre 4.000 et 500.000 et en particulier entre 6.000 et 200.000.

6. Procédé de préparation du polyester-polyuréthanne selon l'une quelconque des revendications précédentes, caractérisé par le fait qu'il consiste à faire réagir, dans un solvant organique, un α,ω-dihydroxypolyester répondant à la formule (IV) suivante : 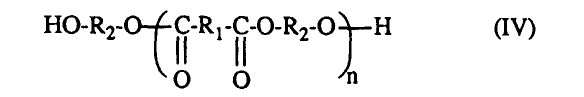 dans laquelle :
R₁, R₂ et n sont tels que définis à la revendication 1,
avec un excès d'un diisocyanate répondant à la formule (V) suivante :
O=C=N-R-N=C=O (V)
dans laquelle :
R est tel que défini à la revendication 1,
puis à coupler les chaînes du polyester-polyuréthanne obtenu précédemment par un diol répondant à la formule (VI) suivante :
HO-A-OH (VI)
dans laquelle :
A est tel que défini à la revendication 1,
à une température comprise entre 40 et 100°C en présence d'un sel d'étain.

7. Procédé selon la revendication 6, caractérisé par le fait que le solvant organique est choisi dans le groupe constitué par l'acétone, la méthyléthylcétone, le tétrahydrofuranne et le 1,2-dichloroéthane.

8. Procédé selon l'une quelconque des revendications 6 et 7, caractérisé par le fait que le sel d'étain est choisi dans le groupe constitué par l'éthyl-2 hexanoate d'étain et le dibutyl dilaurate d'étain.

9. Procédé selon l'une quelconque des revendications 6 à 8, caractérisé par le fait que le diisocyanate de formule (V) est choisi dans le groupe constitué par le diphénylméthane 4,4'-diisocyanate et le méthylène 4,4'-bis-cyclohexyl diisocyanate.

10. Procédé selon l'une quelconque des revendications 6 à 9, caractérisé par le fait que le diol de formule (VI) est choisi dans le groupe constitué par l'acide diméthylol propionique et la N-méthyldiéthanolamine.

11. Procédé selon l'une quelconque des revendications 6 à 10, caractérisé par le fait que l'on fait réagir en outre un coupleur répondant à la formule (VII) suivante :
H-B-X-B'-H (VII)
dans laquelle B, B' et X sont tels que définis à la revendication 4.

12. Procédé selon la revendication 11 caractérisé par le fait que le coupleur de formule (VII) est choisi dans le groupe constitué par le 1,3-diaminopropane et l'éthanolamine.

13. Pseudo-latex stable, caractérisé par le fait qu'il est constitué de particules de polyester-polyuréthanne selon l'une quelconque des revendications 1 à 5, ou obtenu selon les revendications 6 à 12, neutralisé à l'aide d'un agent neutralisant choisi parmi soit une base minérale ou organique lorsque le radical A des motifs de formule (II) est substitué par une fonction acide carboxylique ou sulfonique, soit un acide minéral ou organique lorsque le radical A des motifs de formule (II) est interrompu par un atome d'azote tertiaire, à un taux de neutralisation compris entre 20 et 100 %, le diamètre moyen desdites particules étant compris entre 5 et 300 nm.

14. Pseudo-latex selon la revendication 13, caractérisé par le fait que la base minérale ou organique est choisie dans le groupe constitué par la soude, la potasse, l'ammoniaque, le 2-amino 2-méthyl 1-propanol (AMP), la triéthanolamine, la triisopropanolamine (TIPA), la monoéthanolamine, la diéthanolamine, la tri[(2-hydroxy)1-propyl]amine, la 2-amino 2-méthyl 1,3-propanediol (AMPD), le 2-amino 2-hydroxyméthyl 1,3-propanediol et la lysine.

15. Pseudo-latex selon la revendication 13, caractérisé par le fait que l'acide minéral ou organique est choisi dans le groupe constitué par l'acide chlorhydrique, l'acide lactique, l'acide glycolique et l'acide mandélique.

16. Composition cosmétique, caractérisée par le fait qu'elle contient dans un support cosmétique approprié, un pseudo-latex selon l'une quelconque des revendications 13 à 15.

17. Composition cosmétique selon la revendication 16, caractérisée par le fait que le pseudo-latex est présent en une proportion comprise entre 0,5 et 30 %, et de préférence entre 1 et 25 % en poids par rapport au poids total de la composition.

18. Composition cosmétique selon l'une quelconque des revendications 16 et 17, caractérisée par le fait que le pseudo-latex contient un agent plastifiant en une proportion comprise entre 5 et 50 % en poids par rapport au poids total du pseudo-latex.

19. Composition cosmétique selon l'une quelconque des revendications 16 à 18, caractérisée par le fait qu'elle contient en outre au moins un additif cosmétique choisi dans le groupe constitué par un corps gras, un solvant organique, une silicone, un agent épaississant, un adoucissant, un filtre solaire UV-A ou UV-B ou à bande large, un agent anti-mousse, un agent hydratant, un humectant, un agent durcisseur des ongles, un polymère anionique, non-ionique ou amphotère ou leurs mélanges, un antiperspirant, un agent alcalinisant, un colorant, un pigment et un agent propulseur.

20. Composition cosmétique selon l'une quelconque des revendications 16 à 19, caractérisée par le fait qu'elle constitue une sous-couche pour l'application ultérieure d'un vernis à ongles coloré.

21. Composition cosmétique selon l'une quelconque des revendications 16 à 19, caractérisée par le fait qu'elle constitue une couche de revêtement après l'application préalable d'un vernis à ongles coloré.

22. Composition cosmétique selon l'une quelconque des revendications 16 à 19, caractérisée par le fait qu'elle se présente sous forme d'un vernis à ongles contenant : (i) de 1 à 25 % en poids d'au moins un pseudo-latex tel que revendiqué selon les revendications 13 à 15, (ii) de 0,01 à 5 % en poids d'au moins un agent épaississant, (iii) moins de 3 % en poids d'un pigment, (iv) de 0,1 à 1 % en poids d'au moins un agent mouillant, le reste étant essentiellement constitué par de l'eau.

## Patentansprüche

1. Polyesterpolyurethan, dadurch gekennzeichnet, daß es Einheiten enthält, die den folgenden Formeln (I) und (II) entsprechen: 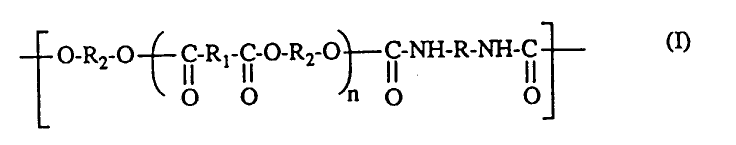 worin
R einen Alkylenrest, Cycloalkylenrest oder einen zweiwertigen aromatischen Rest mit 6 bis 15 Kohlenstoffatomen bedeutet,
n eine ganze Zahl darstellt, so daß das jeweilige Molekulargewicht der repetierenden Einheit zwischen 400 und 5000 beträgt,
R₁ einen zweiwertigen Rest bedeutet, der aus der Gruppe ausgewählt ist, die aus den folgenden Substituenten besteht:
(i) (̵CH₂ 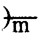 , wobei m eine ganze Zahl zwischen 2 und 12 beträgt, sowie
(ii) 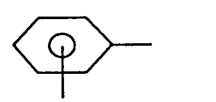 wobei die bewegliche Bindung in der ortho-, meta- oder para-Stellung liegt,
R₂ einen zweiwertigen Rest darstellt, der aus der Gruppe ausgewählt ist, die aus den folgenden Substitutionen besteht: 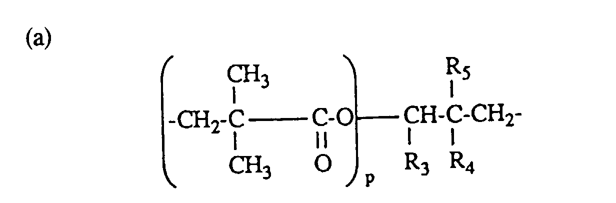 wobei
R₃ ein Wasserstoffatom oder einen verzweigten Alkylrest mit 1 bis 3 Kohlenstoffatomen bedeutet,
R₄ ein Wasserstoffatom oder einen gerad- oder verzweigtkettigen Alkylrest mit 1 bis 4 Kohlenstoffatomen darstellt,
R₅ einen gerad- oder verzweigtkettigen Alkylrest mit 1 bis 4 Kohlenstoffatomen und
p 0 oder 1 bedeuten, 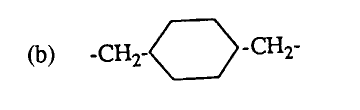 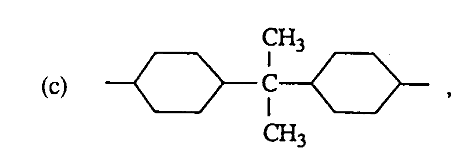 sowie 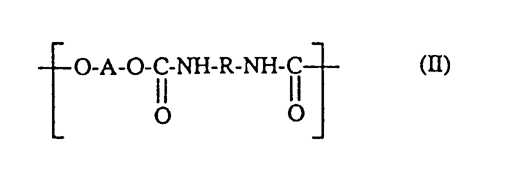 worin
R die in bezug auf die Einheiten gemäß der Formel (I) bezeichneten Bedeutungen aufweist,
A einen Alkylenrest mit 2 bis 20 Kohlenstoffatomen bedeutet, der durch eine Carbonsäure- oder Sulfonsäurefunktion, gegebenenfalls in der Salzform, substituiert ist, oder (in der Kette) durch ein tertiäres Stickstoffatom unterbrochen ist,
wobei das molare Verhältnis zwischen den Einheiten (II) und (I) zwischen 1 und 10, vorzugsweise zwischen 1 und 5, beträgt.

2. Polyesterpolyurethan gemäß Anspruch 1, dadurch gekennzeichnet, daß der Rest R der Einheit gemäß der Formel (I) aus der Gruppe ausgewählt ist, die aus den Gruppen Hexamethylen, 4,4'-Biphenylenmethan, 2,4- und/oder 2,6-Tolylen, 1,5-Naphthylen, p-Phenylen, 4,4'-Methylen-biscyclohexyl und dem von Isophoron abgeleiteten zweiwertigen Rest besteht.

3. Polyesterpolyurethan gemäß einem der vorstehenden Ansprüche, dadurch gekennzeichnet, daß der zweiwertige Rest A der Einheit gemäß der Formel (II) aus der aus den folgenden Substituenten bestehenden Gruppe ausgewählt ist: 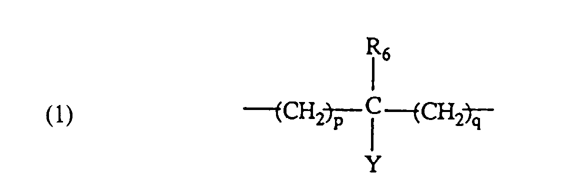 wobei
R₆ einen gerad- oder verzweigtkettigen Alkylrest mit 1 bis 3 Kohlenstoffatomen bedeutet,
Y eine Carbonsäure- oder Sulfonsäuregruppe darstellt, und
p und q, die gleich oder verschieden sind, eine ganze Zahl zwischen 1 und 5 bedeuten,
oder ein Salz der genannten Säuren, 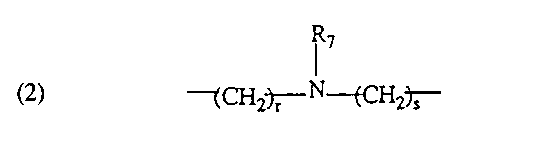 wobei
R₇ einen gerad- oder verzweigtkettigen Alkylrest mit 1 bis 4 Kohlenstoffatomen bedeutet, und
r und s, die gleich oder verschieden sind, eine ganze Zahl zwischen 1 und 10 bedeuten.

4. Polyesterpolyurethan gemäß einem der vorstehenden Ansprüche, dadurch gekennzeichnet, daß es zusätzlich noch Einheiten enthält, die der folgenden Formel (III) entsprechen: 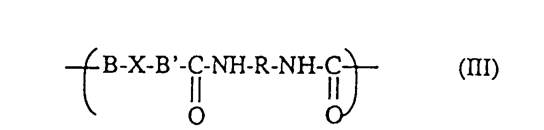 worin
R die in Anspruch 1 definierte Bedeutung aufweist,
B und B', die gleich oder verschieden sind, -O- oder -NH- bedeuten, wobei B und B' nicht gleichzeitig -O- darstellen kann, und
X einen Alkylen- oder Cycloalkylenrest mit 2 bis 12 Kohlenstoffatomen oder einen zweiwertigen aromatischen Rest mit 6 bis 12 Kohlenstoffatomen bedeutet, wobei die jeweilige Einheit in einem Anteil vorhanden ist, daß das Molverhältnis der Summe der Einheiten gemäß den Formeln (II) und (III) sowie der Einheiten gemäß der Formel (I) eine ganze Zahl zwischen 1 und 10, vorzugsweise zwischen 1 und 5, beträgt.

5. Polyesterpolyurethan gemäß einem der vorstehenden Ansprüche, dadurch gekennzeichnet, daß es ein Molekulargewicht besitzt, das durch die sterische Ausschlußchromatographie bestimmt wird und das jeweilige Molekulargewicht zwischen 4 000 und 500 000, insbesondere zwischen 6 000 und 200 000, beträgt.

6. Verfahren zur Herstellung des Polyesterpolyurethans gemäß einem der vorstehenden Ansprüche, dadurch gekennzeichnet, daß es darin besteht, daß in einem organischen Lösungsmittel ein α,ω-Dihydroxypolyester, der der folgenden Formel (IV) entspricht, zur Reaktion gebracht wird: 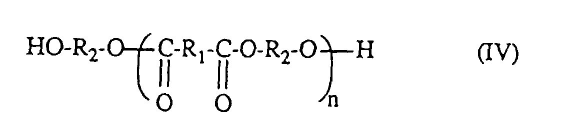 worin
R₁, R₂ und n die in Anspruch 1 angegebene Bedeutung aufweisen,
wobei die vorstehende Verbindung mit einem Überschuß an einem Diisocyanat der folgenden Formel (V) zur Reaktion gebracht wird:
O=C=N-R-N=C=O (V)
worin R die in Anspruch 1 angegebene Bedeutung aufweist,
wonach die Verkupplung der Ketten des vorstehend erhaltenen Polyesterpolyurethans mittels eines Diols der folgenden Formel (VI)
HO-A-OH (V)
worin A die in Anspruch 1 angegebene Bedeutung aufweist,
bei einer Temperatur zwischen 40 °C und 100 °C in Anwesenheit eines Zinnsalzes erfolgt.

7. Verfahren gemäß Anspruch 6, dadurch gekennzeichnet, daß das organische Lösungsmittel aus der aus Aceton, Methylethylketon, Tetrahydrofuran und 1,2-Dichlorethan bestehenden Gruppe ausgewählt wird.

8. Verfahren gemäß einem der Ansprüche 6 und 7, dadurch gekennzeichnet, daß dasZinnsalz aus der aus 2-Ethylhexanoatzinn und Dibutyldilauratzinn bestehenden Gruppe ausgewählt wird.

9. Verfahren gemäß einem der Ansprüche 6 bis 8, dadurch gekennzeichnet, daß dasDiisocyanat gemäß der Formel (V) aus der aus 4,4'-Diphenylmethan-diisocyanat und 4,4'-Methylen-bis-cyclohexyl-diisocyanat bestehenden Gruppe ausgewählt wird.

10. Verfahren gemäß einem der Ansprüche 6 bis 9, dadurch gekennzeichnet, daß das Diol gemäß der Formel (VI) aus der aus Dimethylolpropionsäure und N-Methyldiethanolamin bestehenden Gruppe ausgewählt wird.

11. Verfahren gemäß einem der Ansprüche 6 bis 10, dadurch gekennzeichnet, daß zusätzlich noch ein Kupplungsmittel, das der folgenden Formel (VII) entspricht, zur Reaktion gebracht wird:
H-B-X-B'-H (VII)
worin B, B' und X die in Anspruch 4 angegebenen Bedeutungen aufweisen.

12. Verfahren gemäß Anspruch 11, dadurch gekennzeichnet, daß das Kupplungsmittel gemäß der Formel (VII) aus der aus 1,3-Diaminopropan und Ethanolamin bestehenden Gruppe ausgewählt wird.

13. Stabiler Pseudolatex, dadurch gekennzeichnet, daß er aus Polyesterpolyurethanteilchen gemäß einem der Ansprüche 1 bis 5 besteht, oder gemäß den Ansprüchen 6 bis 12 erhalten wurde und mittels eines Neutralisierungsmittels neutralisiert wurde, das entweder aus einer mineralischen Base oder einer organischen Base im Falle der Substitution des Restes A der Einheiten gemäß der Formel (II) durch eine Carbonsäure- oder Sulfonsäurefunktion oder aus einer Mineralsäure oder organischen Säure im Falle der Unterbrechung des Restes A der Einheiten gemäß der Formel (II) durch ein tertiäres Stickstoffatom ausgewählt wurde, wobei der Neutralisierungsgrad zwischen 20 % und 100 % und der mittlere Durchmesser dieser Teilchen zwischen 5 nm und 300 nm betragen.

14. Pseudolatex gemäß Anspruch 13, dadurch gekennzeichnet, daß die mineralische oder organische Base aus der aus Natriumcarbonat, Kaliumcarbonat, Ammoniak, 2-Amino-2-methyl-1-propanol (AMP), Triethanolamin, Triisopropanolamin (TIPA), Monoethanolamin, Diethanolamin, Tri[(2-hydroxy)-1-propyl]-amin, 2-Amino-2-methyl-1,3-propandiol (AMPD), 2-Amino-2-hydroxymethyl-1,3-propandiol und Lysin bestehenden Gruppe ausgewählt ist.

15. Pseudolatex gemäß Anspruch 13, dadurch gekennzeichnet, daß die Mineralsäure oder organische Säure aus der aus Salzsäure, Milchsäure, Glykolsäure und Mandelsäure bestehenden Gruppe ausgewählt ist.

16. Kosmetische Zubereitung, dadurch gekennzeichnet, daß sie in einem geeigneten kosmetischen Träger einen Pseudolatex gemäß einem der Ansprüche 13 bis 15 enthält.

17. Kosmetische Zubereitung gemäß Anspruch 16, dadurch gekennzeichnet, daß der Peudolatex in einem Mengenverhältnis von 0,5 Gew.-% und 30 Gew.-%, vorzugsweise von 1 Gew.-% und 25 Gew.-%, in bezug auf das Gesamtgewicht der Zusammensetzung vorhanden ist.

18. Kosmetische Zubereitung gemäß einem der Ansprüche 16 und 17, dadurch gekennzeichnet, daß der Pseudolatex einen Weichmacher in einem Mengenverhältnis zwischen 5 Gew.-% und 50 Gew.-% in bezug auf das Gesamtgewicht des Pseudolatex enthält.

19. Kosmetische Zubereitung gemäß einem der Ansprüche 16 bis 18, dadurch gekennzeichnet, daß sie zusätzlich noch ein kosmetisches Additiv enthält, das aus der aus einem Fettkörper, organischen Lösungsmittel, Silikon, Verdickungsmittel, Erweichungsmittel, einer UV-A- oder UV-B-Sonnenfiltersubstanz oder Breit bandsonnenfiltersubstanz, einem Antischaummittel, Hydratisierungsmittel, Feuchthaltemittel, Nagelhärter, anionischen, nichtionischen oder amphoteren Polymer oder deren Gemischen, einem Transpirationshemmer, Alkalisierungsmittel, Farbstoff, Pigment oder einem Treibmittel bestehenden Gruppe ausgewählt ist.

20. Kosmetische Zubereitung gemäß einem der Ansprüche 16 bis 19, dadurch gekennzeichnet, daß sie eine Unterschichtung zur nachfolgenden Applikation eines gefärbten Nagellacks bildet.

21. Kosmetische Zubereitung gemäß einem der Ansprüche 16 bis 19, dadurch gekennzeichnet, daß sie eine Überzugsschicht nach der vorherigen Anwendung eines farbigen Nagellacks bildet.

22. Kosmetische Zubereitung gemäß einem der Ansprüche 16 bis 19, dadurch gekennzeichnet, daß sie in Form eines Nagellacks vorliegt, der folgende Bestandteile enthält:
(i) 1 Gew.-% bis 25 Gew.-% mindestens eines Pseudolatex, wie er gemäß den Ansprüchen 13 bis 15 beansprucht wird,
(ii) 0,01 Gew.-% bis 5 Gew.-% mindestens eines Verdickungsmittels,
(iii) mindestens 3 Gew.-% eines Pigments, und
(iv) 0,1 Gew.-% bis 1 Gew.-% mindestens eines erweichenden Mittels, wobei der Rest im wesentlichen aus Wasser besteht.

## Claims

1. Polyester polyurethane, characterized in that it contains units corresponding to the following formulae (I) and (II): 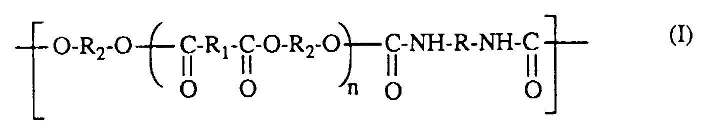 in which:
R represents an alkylene or cycloalkylene radical or a bivalent aromatic radical having from 6 to 15 carbon atoms,
n represents an integer such that the molecular weight of the recurring unit is between 400 and 5,000,
R₁ represents a bivalent radical chosen from the group consisting of:
(i) (̵CH₂ 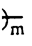 , m being an integer between 2 and 12, and
(ii) 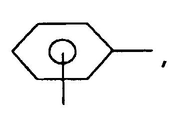 the movable bond being in the ortho, meta or para position,
R₂ represents a bivalent radical chosen from the group consisting of: 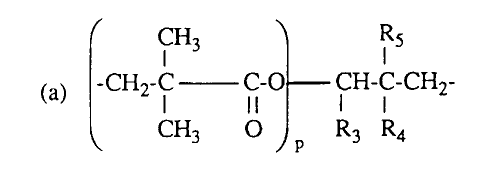
R₃ representing a hydrogen atom or a branched alkyl radical having from 1 to 3 carbon atoms,
R₄ representing a hydrogen atom or a linear or branched alkyl radical having from 1 to 4 carbon atoms,
R₅ representing a linear or branched alkyl radical having from 1 to 4 carbon atoms, and
p being 0 or 1, 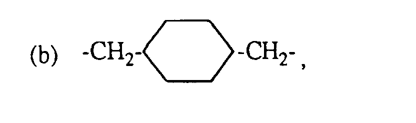 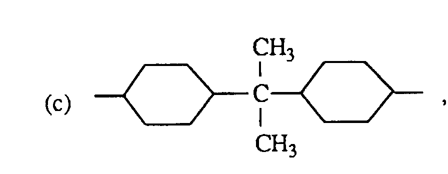 and 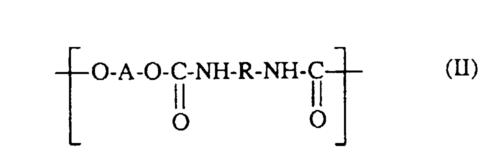 in which:
R is as defined above for the units of formula (I),
A represents an alkylene radical having from 2 to 20 carbon atoms, substituted with a carboxylic or sulphonic acid function, in salified or unsalified form, or interrupted by a tertiary nitrogen atom,
the mole ratio between the units (II) and (I) being between 1 and 10, and preferably between 1 and 5.

2. Polyester polyurethane according to Claim 1, characterized in that the radical R of the unit of formula (I) is chosen from the group consisting of hexamethylene, 4,4'-biphenylenemethane, 2,4- and/or 2,6-tolylene, 1,5-naphthylene, p-phenylene and 4,4'-methylenebis(cyclohexyl) radicals and the bivalent radical derived from isophorone.

3. Polyester polyurethane according to any one of the preceding claims, characterized in that the bivalent radical A of the unit of formula (II) is chosen from the group consisting of: 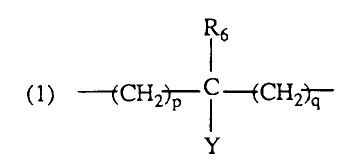
R₆ representing a linear or branched alkyl radical having from 1 to 3 carbon atoms,
Y representing a carboxylic acid or sulphonic acid group, and
p and q, which may be identical or different, representing an integer between 1 and 5, or a salt of the said acids, 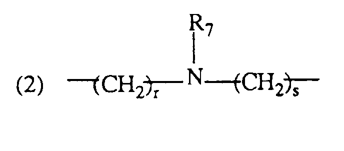
R₇ representing a linear or branched alkyl radical having from 1 to 4 carbon atoms, and
r and s, which may be identical or different, representing an integer between 1 and 10.

4. Polyester polyurethane according to any one of the preceding claims, characterized in that it contains, in addition, units corresponding to the following formula (III) : 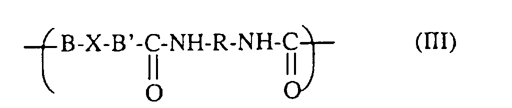 in which:
R is as defined in Claim 1,
B and B', which may be identical or different, represent -O- or -NH-, it not being possible for B and B' simultaneously to represent -O-, and
X represents an alkylene or cycloalkylene radical having from 2 to 12 carbon atoms or a bivalent aromatic radical having from 6 to 12 carbon atoms,
the said unit being present in a proportion such that the mole ratio of the sum of the units of formulae (II) and (III) to the units of formula (I) is an integer between 1 and 10, and preferably between 1 and 5.

5. Polyester polyurethane according to any one of the preceding claims, characterized in that it possesses a molecular weight, measured by stearic exclusion chromatography, of between 4,000 and 500,000, and especially between 6,000 and 200,000.

6. Process for preparing the polyester polyurethane according to any one of the preceding claims, characterized in that it consists in reacting, in an organic solvent, an α,ω-dihydroxy polyester corresponding to the following formula (IV) : 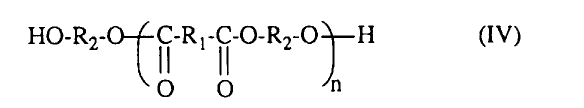 in which:
R₁, R₂ and n are as defined in Claim 1,
with an excess of a diisocyanate corresponding to the following formula (V):
O=C=N-R-N=C=O (V)
in which:
R is as defined in Claim 1,
and then in coupling the chains of the polyester polyurethane obtained above with a diol corresponding to the following formula (VI) :
HO-A-OH (VI)
in which:
A is as defined in Claim 1,
at a temperature of between 40 and 100°C in the presence of a tin salt.

7. Process according to Claim 6, characterized in that the organic solvent is chosen from the group consisting of acetone, methyl ethyl ketone, tetrahydrofuran and 1,2-dichloroethane.

8. Process according to either of Claims 6 and 7, characterized in that the tin salt is chosen from the group consisting of tin 2-ethylhexanoate and dibutyltin dilaurate.

9. Process according to any one of Claims 6 to 8, characterized in that the diisocyanate of formula (V) is chosen from the group consisting of diphenylmethane 4,4'-diisocyanate and 4,4'-methylenebis(cyclohexyl isocyanate).

10. Process according to any one of Claims 6 to 9, characterized in that the diol of formula (VI) is chosen from the group consisting of dimethylolpropionic acid and N-methyldiethanolamine.

11. Process according to any one of Claims 6 to 10, characterized in that a coupler corresponding to the following formula (VII) :
H-B-X-B'-H (VII)
in which B, B' and X are defined as in Claim 4,
is reacted in addition.

12. Process according to Claim 11, characterized in that the coupler of formula (VII) is chosen from the group consisting of 1,3-diaminopropane and ethanolamine.

13. Stable pseudolatex, characterized in that it consists of particles of polyester polyurethane according to any one of Claims 1 to 5, or obtained according to Claims 6 to 12, neutralized using a neutralizing agent chosen from either an inorganic or organic base when the radical A of the units of formula (II) is substituted with a carboxylic or sulphonic acid function, or an inorganic or organic acid when the radical A of the units of formula (II) is interrupted by a tertiary nitrogen atom, to a degree of neutralization of between 20 and 100 %, the average diameter of the said particles being between 5 and 300 nm.

14. Pseudolatex according to Claim 13, characterized in that the inorganic or organic base is chosen from the group consisting of sodium hydroxide, potassium hydroxide, ammonia solution, 2-amino-2-methyl-1-propanol (AMP), triethanolamine, triisopropanolamine (TIPA), monoethanolamine, diethanolamine, tris(2-hydroxy-1-propyl)amine, 2-amino-2-methyl-1,3-propanediol (AMPD), 2-amino-2-hydroxymethyl-1,3-propanediol and lysine.

15. Pseudolatex according to Claim 13, characterized in that the inorganic or organic acid is chosen from the group consisting of hydrochloric acid, lactic acid, glycolic acid and mandelic acid.

16. Cosmetic composition, characterized in that it contains a pseudolatex according to any one of Claims 13 to 15, in a suitable cosmetic carrier.

17. Cosmetic composition according to Claim 16, characterized in that the pseudolatex is present in a proportion of between 0.5 and 30 %, and preferably between 1 and 25 %, by weight relative to the total weight of the composition.

18. Cosmetic composition according to either of Claims 16 and 17, characterized in that the pseudolatex contains a plasticizing agent in a proportion of between 5 and 50 % by weight relative to the total weight of the pseudolatex.

19. Cosmetic composition according to any one of Claims 16 to 18, characterized in that it contains, in addition, at least one cosmetic additive chosen from the group consisting of a fat, an organic solvent, a silicone, a thickening agent, an emollient, a UV-A or UV-B or broad-band sunscreen agent, an antifoaming agent, a hydrating agent, a humectant, a nail hardener, an anionic, nonionic or amphoteric polymer or mixtures thereof, an antiperspirant, an alkalinizing agent, a colorant, a pigment and a propellent agent.

20. Cosmetic composition according to any one of Claims 16 to 19, characterized in that it constitutes a base coat for the subsequent application of a coloured nail varnish.

21. Cosmetic composition according to any one of Claims 16 to 19, characterized in that it constitutes a top coat after the prior application of a coloured nail varnish.

22. Cosmetic composition according to any one of Claims 16 to 19, characterized in that it takes the form of a nail varnish containing: (i) from 1 to 25 % by weight of at least one pseudolatex as claimed according to Claims 13 to 15, (ii) from 0.01 to 5 % by weight of at least one thickening agent, (iii) less than 3 % by weight of a pigment, (iv) from 0.1 to 1 % by weight of at least one wetting agent, the remainder consisting essentially of water.
